# EUROPÄISCHE PATENTSCHRIFT

(11) **EP 3 030 281 B1**
(45) Veröffentlichungstag und Bekanntmachung des Hinweises auf die Patenterteilung: **01.11.2017**
(21) Anmeldenummer: 14747844.0
(22) Anmeldetag: 06.08.2014
(51) Int. Cl.: A61M 1/14, A61M 1/34, B29C 65/16, B23K 26/20, B29C 65/00

(54) **KASSETTENMODUL**
CASSETTE MODULE
MODULE CASSETTE

(30) Priorität: 09.08.2013 DE 102013013415
(43) Veröffentlichungstag der Anmeldung: 15.06.2016
(73) Patentinhaber: Fresenius Medical Care Deutschland GmbH, 61352 Bad Homburg (DE)
(72) Erfinder: KREBER, Stefan, 66113 Saarbrücken (DE); WEIS, Manfred, 66606 St. Wendel (DE); WENKE, Marina, 66606 St. Wendel (DE); LEICK, Lothar, 66603 Merzig (DE)
(74) Vertreter: Herrmann, Uwe
(86) Internationale Anmeldenummer: PCT/EP2014/002162
(87) Internationale Veröffentlichungsnummer: WO 2015/018519

(56) Entgegenhaltungen:
- WO-A1-2008/106440
- WO-A2-2012/087798
- US-A1- 2007 278 155
- US-A1- 2010 274 168
- US-A1- 2011 028 902
- US-A1- 2011 200 791
- US-A1- 2012 167 673

## Beschreibung

Die vorliegende Erfindung betrifft ein Kassettenmodul zur Steuerung von Fluidströmen, insbesondere zur Verwendung in Blutbehandlungssystemen oder Infusionssystemen.
Aus dem Stand der Technik sind Kassettenmodule beispielsweise für den Einsatz bei Dialysegeräten, insbesondere bei der Hämodialyse oder Peritonealdialyse bekannt. Diese Kassettenmodule haben die Aufgabe, im Rahmen der Behandlung Flüssigkeitsströme, insbesondere Blut, Dialysat oder sonstige behandlungsrelevante Flüssigkeiten mit Hilfe von integrierten Funktionseinheiten steuern bzw. fördern zu können. Solche Funktionseinheiten sind beispielsweise Ventile, Pumpen, Kanäle etc. Durch diese lassen sich die Ströme von Blut, Dialysat und anderen behandlungsrelevanten Flüssigkeiten in der gewünschten Weise steuern.
Kassettenmodule können aufgrund der verwendeten vergleichsweise kostengünstigen Kunststoffe in Massenfertigung hergestellt werden und finden häufig als Einwegartikel Verwendung.
Die genannten Ventil- und Pumpfunktionen sind in Kassettenmodulen aus dem Stand der Technik bekannt. In Kassettenkörpern können Strömungskanäle und/oder Öffnungen ausgebildet sein, die mit einer bewegbaren Membran in Kontakt gebracht werden können, um auf diese Weise die genannte Funktion bereitstellen zu können. So ist es beispielsweise denkbar, die Membran zum Pumpen einer Flüssigkeit zu verwenden sowie auch durch die Membran je nach deren Position eine Öffnung etc. zu überdecken und so eine Ventilfunktion bereitzustellen.
Die Auslenkung der Membran selbst kann beispielsweise durch eine pneumatische Aktuierung erfolgen.

Aus der EP 0 129 554 B1 ist ein Kassettenmodul bekannt, das aus zwei Platten besteht, zwischen denen eine bewegbare Membran angeordnet ist. Diese wird durch eine pneumatische Aktuierung angesteuert. Dazu ist eine der Platten des Kassettenmoduls als Aktorplatte ausgeführt, die mit Kanälen und Kammern ausgeführt ist, in denen je nach Bedarf Überdruck oder Unterdruck erzeugt werden kann.
Die US 5,350,357 offenbart ein Kassettenmodul, das über ein maschinenseitiges pneumatisches Aktormodul betrieben werden kann. Das Kassettenmodul umfasst einen Grundkörper, der auf seiner Vorder- und Rückseite mit einer Membran überzogen ist. Die pneumatische Ansteuerung und damit die Steuerung der Flüssigkeitsströme innerhalb des Kassettenmoduls erfolgt über die pneumatische Auslenkung einer der Membranen.
Die WO 2008/106440 A1 offenbart schließlich eine Kassette, die aus drei Platten besteht, wobei die Flüssigkeitswege innerhalb des Kassettenmoduls im Wesentlichen auf der mittleren Platte angeordnet sind, die zwischen den beiden außenliegenden Platten angeordnet ist. Über einzelne Membranen können die erforderlichen Pump- und Ventilfunktionen erzeugt werden. Die Auslenkung der Membranen erfolgt über pneumatische Anschlüsse, die in einer der Platten angeordnet sind. US2007/0278155 A1 offenbart ein weiteres Kassettenmodul aus dem Stand der Technik. Die US 2011/028902 A1 offenbart ein Kassettenmodul, das die Merkmale des Oberbegriffs des Anspruchs 1 aufweist. Bei aus dem Stand der Technik bekannten Kassettenmodulen ist die zu fördernde Flüssigkeit von der Antriebsseite, d.h. von der Pneumatikseite durch die genannte Membran getrennt.
Die Auslenkung der Membranen erfolgt über pneumatische Anschlüsse, die in einer der Platten angeordnet sind.

Der vorliegenden Erfindung liegt die Aufgabe zugrunde, ein Kassettenmodul dahingehend weiterzubilden, dass eine besonders zuverlässige Betätigung der Mittel zur Strömungsführung des Fluids erfolgt.

Diese Aufgabe wird durch ein Kassettenmodul mit den Merkmalen des Anspruchs 1 gelöst.

Danach ist vorgesehen, dass zwischen den Membranen wenigstens ein Betätigungselement angeordnet ist, mittels dessen direkt oder indirekt auf die Mittel zur Strömungsführung eingewirkt werden kann.

Handelt es sich bei den Mitteln zur Strömungsführung beispielsweise um eine Öffnung bzw. um ein Ventil, kann durch das genannte Betätigungselement, das sich zwischen den beiden Membranen befindet, besonders zuverlässig z. B. ein Druck auf diese Öffnung ausgeübt und somit das Ventil geschlossen werden.

Die Ansteuerung bzw. Aktuierung einer oder beider Membranen kann beispielsweise pneumatisch oder auch durch eine Flüssigkeit bzw. hydraulisch erfolgen.

Eine zuverlässige Kraftübertragung von einer Membran auf die andere Membran erfolgt mittels des genannten Betätigungselementes, das beispielsweise als Stößelscheibe, Platte etc. ausgeführt sein kann, die den Druck, der auf eine Membran, z. B. pneumatisch ausgeübt wird, vorzugsweise flächig auf die zweite Membran überträgt. Dadurch wird ein besonders zuverlässiges Schließen beispielsweise eines Kanals, einer Öffnung etc. bewirkt.

Durch das Vorhandensein zweier Membranen ergibt sich darüber hinaus der Vorteil, dass das Reißen einer Membran nicht zu einer sofortigen Kontamination der in der Kassette strömenden Behandlungsflüssigkeit oder eines sonstigen Fluids durch das Aktormedium, wie z.B. Pressluft oder ein hydraulisches Medium führt.

Auch ein Austreten der geförderten Flüssigkeit bzw. des geförderten Gases kann durch zwei Membranen entsprechend verhindert werden.

Durch die zwischen den zwei Membranen liegende Stößelscheibe oder sonstiges Betätigungselement wird die durch die Membran ausgeübte Funktion, beispielsweise eine Ventilfunktion schneller und präziser ausgeübt, was höhere Taktraten der Ventilschaltungen zulässt.

Ein nicht von der Erfindung umfasster Gegenstand betrifft des Weiteren ein Kassettenmodul zur Steuerung von Fluidströmen, insbesondere zur Verwendung in Blutbehandlungssystemen oder Infusionssystemen, wobei das Kassettenmodul wenigstens einen Grundkörper mit Mitteln zur Strömungsführung wenigstens eines Fluidstroms und wenigstens eine Membran umfasst, die zumindest abschnittsweise unmittelbar oder mittelbar mit dem Grundkörper in Verbindung steht, wobei die Membran einen einschichtigen Aufbau, dessen Schicht aus einem Laserlicht absorbierenden Material besteht oder dieses aufweist, oder einen mehrschichtigen Aufbau aufweist, der wenigstens eine das Laserlicht transmittierende und wenigstens eine das Laserlicht absorbierende (vorzugsweise äußere) Schicht aufweist, wobei die das Laserlicht absorbierende äußere Schicht den Verbindungsbereich der Membran zu wenigstens einer weiteren Komponente des Kassettenmoduls bildet.

Denkbar ist es, dass die Membran zwei äußere, laserlichtabsorbierende Schichten zur Bindung an zwei Funktionsschichten aufweist. Auch ist der Einsatz einer monoschichtigen Membran mit einem Laserabsorber zur Bindung an ein oder zwei Funktionsschichten möglich und von der Erfindung mit umfasst.

Unter einer das Laserlicht absorbierenden Schicht wird eine Schicht verstanden, die eine größere Absorption für das Laserlicht aufweist als die das Laserlicht transmittierende Schicht. Umgekehrt weist die das Laserlicht transmittierende Schicht eine geringere Absorption für das Laserlicht auf, als die das Laserlicht absorbierende Schicht. Die Begriffe "absorbierende Schicht" und "transmittierende Schicht" sind somit nicht zwingend dahingehend zu verstehen, dass die absorbierende Schicht einen Absorptionsgrad von 100 % und die transmittierende Schicht einen Absorptionsgrad von 0 % hat, wenngleich auch eine solche Ausführung von der Erfindung umfass ist. Die Begriffe verdeutlichen insbesondere ein unterschiedliches Absorptionsverhalten der Schichten und damit auch eine unterschiedliche Erwärmung bei der Beaufschlagung mit Laserlicht. Die Werte von 100% und 0% Absorptionsgrad sind Grenzwerte, die nur nach physikalischer Gesetzmäßigkeit möglich sind. Grundsätzlich wird man im Allgemeinen im transparenten Teil der Schicht eine minimale Absorption und im opaken Teil der Schicht eine bestimmte Transmission beobachten können.

Eine zuverlässige Verbindung zwischen der Membran und wenigstens einer weiteren Komponente des Kassettenmoduls, beispielsweise dem Grundkörper wird dadurch erreicht, dass somit wenigstens eine mehrschichtige Membran eingesetzt wird. Durch das Aufbringen von Laserlicht wird die das Laserlicht absorbierende Schicht erhitzt, so dass es zu einem "Verschweißen" der Membran mit wenigstens einem weiteren Teil des Kassettenmoduls kommt. Die das Laserlicht transmittierende Schicht wird hingegen nicht oder kaum bzw. weniger stark erwärmt und dient insbesondere zur Gewährleistung der mechanischen Stabilität der Membran.

Da auf der Fluidseite, d.h. auf der Seite des Fluids, dessen Fluss gesteuert werden soll, auch Unterdruck herrschen kann, ist es notwendig, die Aktorseite und die Fluidseite des Ventils oder des sonstigen Kassettenkörpers miteinander zu koppeln, da sonst bei einer geschlossenem Ventil und anliegendem Unterdruck auf der Fluidseite der Druchlass durch Anlegen von Unterdruck nicht mehr geöffnet werden könnte. Werden die beiden Membran auf ihren zugewandten Seiten miteinander verschweißt, kann dieses Problem behoben werden.

Die Begriffe "Unterdruck" und "Überdruck" sind nicht nur als relative Werte zum Atmosphärendruck, sondern auch als Werte relativ zueinander zu verstehen. Dies bedeutet, dass beispielsweise "Unterdruck" nicht zwingenderweise Vakuum bedeuten muss, wenngleich dies möglich ist.

Das Verfahren des Durchstrahlschweißens mittels eines Lasers bringt den Vorteil mit sich, dass die Folie auch noch dann erhitzt werden kann, wenn sie bereits zwischen zwei zu verbindenden Teilen angeordnet ist, d. h. von außen nicht mehr direkt zugänglich ist.

In einer weiteren Ausgestaltung der Erfindung können die beiden oben genannten Gedanken kombiniert vorliegen.

Bei der weiteren Komponente, mit der die Membran verbunden ist, kann es sich beispielsweise um den Grundkörper oder um einen Teil des Grundkörpers des Kassettenmoduls oder auch um eine weitere Membran oder auch um das oben genannte Betätigungselement, wie beispielsweise die Stößelscheibe handeln.

Weiterhin kann vorgesehen sein, dass die Membran einen zumindest dreischichtigen Aufbau aufweist, wobei wenigstens zwei das Laserlicht absorbierende Schichten (vorzugsweise als außenliegende Schichten) und wenigstens eine dazwischen angeordnete, das Laserlicht transmittierende Schicht vorhanden ist.

Weiterhin ist vorgesehen, dass das Kassettenmodul wenigstens eine erste Funktionsschicht und wenigstens eine zweite Funktionsschicht aufweist, wobei die erste Funktionsschicht Mittel zur Strömungsführung wenigstens eines Fluidstroms, insbesondere wenigstens eines Flüssigkeitsstroms aufweist und wobei die zweite Funktionsschicht zwischen den beiden Membranen angeordnet ist.

Zudem ist nach der Erfindung vorgesehen, dass das Kassettenmodul eine dritte Funktionsschicht aufweist, die Mittel zur Aktuierung einer oder beider Membranen aufweist.

Die dritte Funktionsschicht kann beispielsweise Kammern, Kanäle oder dergleichen aufweisen, über die über ein pneumatisches oder hydraulisches Betätigen wenigstens eine der Membranen ausgelenkt werden kann.

Vorzugsweise handelt es sich bei den Mitteln zur Aktuierung wenigstens einer der Membranen um pneumatische Mittel, insbesondere um Kammern oder Kanäle, in denen Über- oder Unterdruck herrscht oder erzeugbar ist.

In einer weiteren Ausgestaltung der Erfindung ist vorgesehen, dass die Mittel zur Strömungsführung eines Fluidstroms, wie eines Flüssigkeitsstroms oder eines Gasstroms einen oder mehrere Kanäle, Ventile und Pumpabschnitte aufweisen.

Weiterhin kann vorgesehen sein, dass die genannten Funktionsschichten aus einem biegesteifen Material, vorzugsweise aus Kunststoff bestehen. Die genannte Membran ist flexibel und vorzugsweise elastisch.

Im Sinne der Erfindung ist es nicht zwingend notwendig, dass die Membran flächig zwischen den Funktionsschichten angeordnet ist, wenngleich eine solche Ausführungsform ebenfalls umfasst ist. Die Membran kann auch abschnittsweise auf jeweilige Aktivierungselemente der Funktionsschichten begrenzt angeordnet sein.

Das Kassettenmodul ist vorzugsweise als Einwegartikel ausgeführt.

Die vorliegende Erfindung betrifft des Weiteren eine Maschine, insbesondere ein Blutbehandlungs- oder Infusionsmaschine, wie beispielsweise ein Hämodialysegerät oder ein Peritonealdialysegerät mit wenigstens einer Aufnahme, in der sich wenigstens ein Kassettenmodul gemäß einem der Ansprüche 1 bis 11 befindet.

Die vorliegende Erfindung betrifft des Weiteren ein Verfahren zur Herstellung eines Kassettenmoduls gemäß einem der Ansprüche 1 bis 6, wobei die Verbindung der Membran zu wenigstens einer weiteren Komponente des Kassettenmoduls mittels Durchstrahlschweißen mit einem Laser erfolgt, wobei die Erwärmung der wenigstens einen das Laserlicht absorbierenden Schicht der Membran erfolgt.
Dabei ist vorzugsweise vorgesehen, dass sich die Membran in ihrem in dem Grundkörper eingesetzten Zustand befindet, wenn das Durchstrahlschweißen durchgeführt wird. Um dies zu ermöglichen, ist das Material des Grundkörpers für das Laserlicht in diesem Fall durchlässig.
Weitere Einzelheiten und Vorteile der Erfindung werden anhand eines in der Zeichnung dargestellten Ausführungsbeispiels näher erläutert. Es zeigen:
- Figur 1:: eine Schnittansicht durch ein Kassettenmodul gemäß der vorliegenden Erfindung,
- Figur 2:: eine Schnittansicht durch eine Membran, wie sie in dem erfindungsgemäßen Kassettenmodul zum Einsatz kommen kann und
- Figur 3:: eine weitere Schnittansicht durch ein erfindungsgemäßes Kassettenmodul.

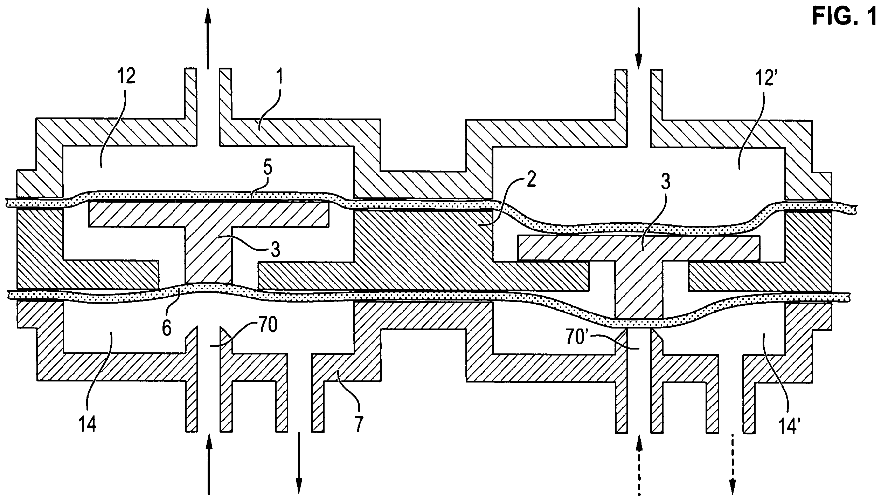

Figur 1 zeigt eine Längsschnittansicht durch ein erfindungsgemäßes Kassettenmodul.
Mit dem Bezugszeichen 7 ist die erste Funktionsschicht und mit dem Bezugszeichen 2 die zweite Funktionsschicht gekennzeichnet.
Das Bezugszeichen 1 kennzeichnet die dritte Funktionsschicht.

Wie dies aus der Figur hervorgeht, befindet sich die zweite Funktionsschicht 2 zwischen der ersten Funktionsschicht 7 und der dritten Funktionsschicht 1.

Des Weiteren befindet sich eine erste Membran 6 zwischen der zweiten Funktionsschicht 2 und der ersten Funktionsschicht 7 und eine zweite Membran 5 zwischen der dritten Funktionsschicht 1 und der zweiten Funktionsschicht 2.

Wie dies aus der Figur weiter hervorgeht, befindet sich zwischen den Membranen 5, 6 in beiden dargestellten Kammern je ein Stößel bzw. je eine Stößelplatte 3, die gleichsam als Ventil zum Verschließen oder Freigeben einer Öffnung 70, 70' in der ersten Funktionsschicht 7 dient.

Wie dies weiter aus der Figur hervorgeht, befinden sich in der ersten Funktionsschicht 7 Mittel zur Strömungsführung wenigstens eines Flüssigkeitsstromes, wie beispielsweise von Blut, Dialysat, einer Infusionslösung etc., wobei in dem hier dargestellten Ausführungsbeispiel die Mittel in Form eines Ventils ausgebildet sind, das eine Durchströmung der Kammern 14, 14' des Kassettenmoduls ermöglicht oder verhindert.

Wie dies aus der Figur weiter hervorgeht, weist der Ventilblock bzw. das als Ventilblock ausgeführte Kassettenmodul zwei solcher Ventile auf, wobei das Ventil mit der Öffnung 70 in der links dargestellten Kammer 14 des Kassettenmoduls geöffnet und das Ventil mit der Öffnung 70' in der rechts dargestellten Kammer 14' des Kassettenmoduls geschlossen ist.

Das Öffnen des Ventils erfolgt dadurch, dass in der Pneumatikkammer 12 wie durch einen Pfeil dargestellt Unterdruck erzeugt wird. Dies führt dazu, dass die zweite Membran 5 und aufgrund des zwischen den Membranen herrschenden Vakuums auch die erste Membran 6 sowie auch die Stößelplatte 3 nach oben bewegt wird, wodurch die Öffnung 70 durch die Membran 6 freigegeben wird. Dadurch wird ein Fluidstrom durch die Fluidkammer 14 ermöglicht.

In der rechts dargestellten Kammer 12' des Kassettenmoduls wird hingegen ein Überdruck erzeugt, wie dies durch dargestellten Pfeil symbolisiert ist, der das Einströmen eines Pneumatikfluids, insbesondere von Pressluft symbolisiert. Dadurch werden die beiden Membranen 5, 6 sowie auch die rechts dargestellte Stößelplatte 3 nach unten gedrückt und mittels der ersten Membran 6 die Öffnung 70' verschlossen. Die Kammer 14' ist somit nicht von einem Fluid durchströmbar, wie dies durch die gestrichelten Pfeile angedeutet wird.

Wie bereits oben ausgeführt bedeuten die Begriffe Überdruck und Unterdruck nicht zwingenderweise, dass ein Überdruck bzw. Unterdruck relativ zum Atmosphärendruck herrschen muss, wenngleich auch eine solche Ausführungsform mit umfasst ist. Sie können auch relative Werte zueinander bedeuten.

So ist es beispielsweise denkbar, dass ein Unterdruck in der Kammer 12 nicht nur bedeutet, dass mit einer Pumpe Vakuum in der Kammer 12 angelegt wird. Es kann auch bedeuten, dass durch den Betrieb der Kassette eine Flüssigkeit in die Kammer 14 eingeströmt wird, ohne dass in der Kammer 12 dagegen aktuiert wird. Die Membranen 5, 6 werden dann ausgelinkt und die Kammer 12 stellt gegenüber der Kammer 14 die Unterdruckseite dar, ohne dass aktiv ein Vakuum in der Kammer 12 angelegt wird.

Denkbar ist es, dass der Druck in der Funktionsschicht 2 geringer ist als in den Schichten 1 und 14.

Bei dem in der Figur dargestellten Kassettenmodul handelt es sich um einen Ventilblock. Grundsätzlich sind auch beliebige andere Funktionalitäten, wie beispielsweise eine Pumpfunktion oder dergleichen von der Erfindung mitumfasst.

Die Funktionsschichten 1, 2, 7 bestehen aus einem biegesteifem Kunststoff, d. h. aus einem formstabilen Kunststoff, wohingegen die Membranen 5, 6 als flexible, vorzugsweise elastische Membranen ausgeführt sind, damit die gewünschte Strömungsführung eingestellt werden kann.

Aus der Figur ist weiter ersichtlich, dass sowohl die erste Funktionsschicht 7 als auch die dritte Funktionsschicht 1 Aufweitungen aufweisen, die Kammern 12, 14, 12', 14' bilden, die einerseits zur Ansteuerung der Membranen über ein Aktormedium, wie z.B. Pressluft und andererseits zur Steuerung eines Fluidstroms, wie beispielsweise von Blut, Dialysat, Infusat etc. dienen.

Das dargestellte Kassettenmodul ist selbstverständlich nicht nur zur Steuerung eines Flüssigkeitsstroms geeignet, sondern auch beispielsweise um einen Gasstrom zu steuern.

Auch die Ansteuerung über die Kammern 12, 12' muss nicht zwingend mit einem Gas bzw. Pressluft erfolgen, sondern kann ebenfalls über eine Flüssigkeit bzw. hydraulisch erfolgen.

Durch den Überdruck bzw. Unterdruck in den Kammern 12, 12' wird das dargestellte Ventil bzw. die Stößelplatte 3 in die ein oder andere Endlage gebracht und verschließt oder öffnet je nach seiner momentanen Position mit seiner der Pneumatik oder Hydraulik entgegengesetzten Seite einen Fluidkreislauf bzw. die Öffnung 70, 70'.

Die Endlagen können beispielsweise durch Flächen der Funktionsschichten, wie beispielsweise durch die zweite Funktionsschicht gebildet werden. In der rechten Kammer steht der plattenförmige Bereich der Stößelplatte an der zweiten Funktionsschicht an, so dass dadurch eine Endlage definiert ist.

Durch das Vorhandensein zweier Membranen 5, 6 wird der Ansteuerungskreislauf, beispielsweise der Pneumatikkreislauf und der Fluidkreislauf, beispielsweise ein Kreislauf zur Förderung von Blut, Dialysat, etc. zuverlässig getrennt, so dass bei dem Reißen einer Membran keine Kontamination auftreten kann.

Die Membran 5 steht vorzugsweise unmittelbar mit den Funktionsschichten 1 und 2 und die Membran steht vorzugsweise unmittelbar mit den Funktionsschichten 2 und 7 in Verbindung.

An dieser Stelle wird darauf hingewiesen, dass der Begriff "Membran" weit zu fassen ist und jedes flächige und bewegbare Material umfasst, das die genannten Funktionen durchführen kann. Die Membranen können beispielsweise als Folien ausgebildet sein.

Sie sind vorzugsweise für das Fluid sowie auch für das Aktormedium (wie z.B. Pressluft, Hydraulikmedium etc.) undurchlässig. Sie sind des Weiteren vorzugsweise weitgehend gasundurchlässig.

Durch die zwischen den Membranen 5, 6 befindliche Stößelscheibe 3 erfolgt die durch die zweite Membran 5 ausgeübte Bewegung bzw. Ventilfunktion schneller und präziser. Dies lässt höhere Taktraten der Ventilschaltung zu, was wünschenswert ist.

Eine mögliche Realisierung der Kopplung zwischen dem Pneumatik- und dem Fluidkreis besteht darin, die beiden Membranen 5, 6 jeweils mit den ihnen zugewandten Seiten zu verschweißen. Auch müssen die beteiligten Gehäuseteile, d. h. die genannten Funktionsschichten miteinander verbunden werden.

Eine kostengünstige als auch technisch einfache Realisierung dieser Verbindungsaufgaben besteht darin, dass wenigstens eine oder beide der in Figur 1 dargestellten Membranen 5, 6 partiell erhitzt wird und damit die angrenzenden Hartkomponenten miteinander verschweißt werden. Die Membranen dienen somit als Verbindungsmittel für die Funktionsschichten.

Die Membran kann dabei weiterhin die für die Ausführungsform gemäß Figur 1 erforderliche Ventil- bzw. Dichtfunktion erfüllen.

Um eine übermäßige Erwärmung zum Zwecke des Schweißens der Membran zu verhindern, weist die Membran wenigstens eine Schicht auf, die das Laserlicht transmittiert.

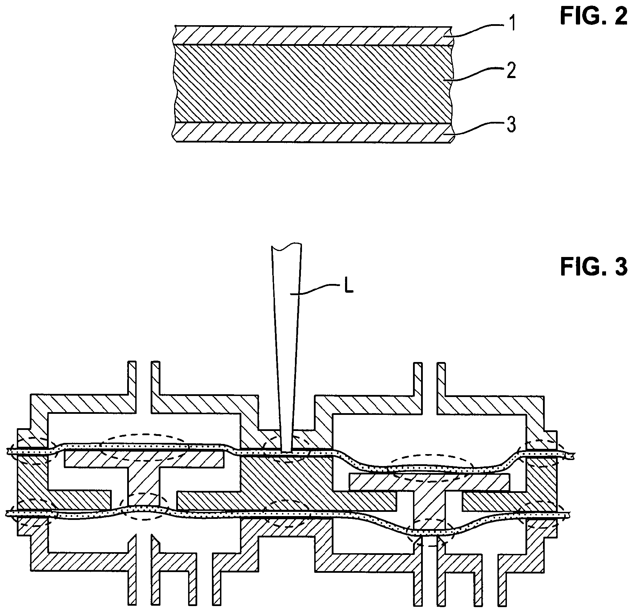

Die Wellenlänge des Lasers kann so gewählt werden, dass das verwendete Kunststoffmaterial der Funktionsschichten für dieses Licht durchlässig ist, während die in Figur 2 gezeigten Schichten 1 und 3 der Membran das Laserlicht absorbierende Anteile aufweist und somit beim Durchstrahlen erwärmt werden.

Bevorzugt wird im Rahmen des erfindungsgemäßen Verfahrens ein Nd:YAG-Laser eingesetzt.

Als Absorber bzw. absorbierendes Material kann beispielsweise Lumogen IR 788 BASF eingesetzt werden, der im Infrarotbereich absorbiert, sodass die damit versehenen Bauteile im Wellenlängenbereich des sichtbaren Lichts weitgehend transparent sind. Vorzugsweise wird der Absorber mit einer Konzentration von 100ppm in das Membranmaterial eingemischt.

Figur 2 zeigt einen exemplarischen Aufbau dieser Membran 5, 6 und verdeutlicht, dass die Membran zwei Deckschichten 1, 3 aufweist, die das Laserlicht absorbierend ausgeführt sind und eine dazwischen liegende Mittelschicht 2, die einen geringeren Absorptionsgrad für das Laserlicht aufweist und damit nicht oder geringfügiger erwärmt wird als die Schichten 1, 3.

Aufgrund der Tatsache, dass die Membran auf ihren Außenseiten nur dünne Schichten 1, 3 aus einem Laserlicht absorbierenden Material aufweist, wird auch nur dort, d. h. an Kontaktstelle zu den zu schweißenden Hartteilen bzw. Komponenten des Grundkörpers bzw. Funktionsschichten eine hohe Temperatur erzeugt, wohingegen die dargestellte Mittelschicht 2 nicht oder nur wenig erwärmt wird und daher die Stabilität des Aufbaus und die plastischen und elastischen Eigenschaften der Membran weitergehend erhalten bleiben.

Durch die für das verwendete Laserlicht transparenten Materialien des Grundkörpers, d. h. insbesondere Funktionsschichten kann ein Schweißen im Inneren des Gesamtaufbaus erfolgen, d. h. ein Schweißen kann erfolgen, nachdem die wenigstens eine Membran bereits eingelegt wurde.

Figur 3 zeigt markiert durch eingekreiste Bereiche die Schweißstellen, an denen die Membran mit wenigstens einer weiteren Komponente durch das erfindungsgemäße Verfahren verbunden ist. Mit dem Bezugszeichen L ist der Laserstrahl gekennzeichnet. Die Verbindung kann zu der Stößelscheibe, zu dem Grundkörper bzw. Funktionsschicht und/oder zu einer weiteren Membran bestehen.

Durch das Schweißen mittels Laser können sowohl einzelne Schweißpunkte gesetzt werden, als auch dünne Bahnen, z. B. zum Einschließen der zu dichtenden Areale gezogen werden. Durch das gezielte Einbringen der Schweißenergie nur auf die benötigten Materialmengen kann der Wärmeeintrag und damit die Belastung des zu schweißenden Kassettenmoduls sehr gering gehalten werden.

## Patentansprüche

1. Kassettenmodul zur Steuerung von Fluidströmen und zur Verwendung in Blutbehandlungssystemen oder Infusionssystemen, umfassend:
einen Grundkörper mit Mitteln zur Strömungsführung (70, 70') eines Fluidstroms, insbesondere eines Flüssigkeitsstroms, und
zwei Membranen (5, 6), die zumindest abschnittsweise mit dem Grundkörper in Verbindung stehen, wobei
zwischen den Membranen (5, 6) ein Betätigungselement (3) angeordnet ist, das dazu ausgelegt ist, auf die Mittel zur Strömungsführung (70, 70') einzuwirken, und
das Kassettenmodul eine erste Funktionsschicht (7), die Mittel zur Strömungsführung (70, 70') eines Fluidstroms umfasst, und eine zweite Funktionsschicht (2), die zwischen den beiden Membranen (5, 6) angeordnet ist, aufweist,
**dadurch gekennzeichnet, dass**
das Kassettenmodul eine dritte Funktionsschicht (1) aufweist, die Mittel zur Aktuierung (12, 12') einer oder beider Membranen (5, 6) aufweist.

2. Kassettenmodul nach Anspruch 1, **dadurch gekennzeichnet, dass** die Mittel zur Aktuierung (12, 12') der Membran (5, 6) pneumatische oder hydraulische Mittel sind, insbesondere Kammern oder Kanäle, in denen Über- oder Unterdruck herrscht oder erzeugbar ist.

3. Kassettenmodul nach einem der vorhergehenden Ansprüche, **dadurch gekennzeichnet, dass** die Mittel zur Strömungsführung (70, 70') eines Fluidstroms einen oder mehrere Kanäle, Ventil- und Pumpabschnitte aufweist.

4. Kassettenmodul nach einem der vorhergehenden Ansprüche, **dadurch gekennzeichnet, dass** wenigstens eine, mehrere oder alle Funktionsschichten (7, 2, 1) aus einem biegesteifen Material, vorzugsweise aus Kunststoff bestehen.

5. Kassettenmodul nach einem der vorhergehenden Ansprüche, **dadurch gekennzeichnet, dass** es sich bei der oder den Membranen (5, 6) um elastische Membranen (5, 6) handelt.

6. Blutbehandlungs- oder Infusionsmaschine mit einer Aufnahme, in der sich ein Kassettenmodul gemäß einem der Ansprüche 1 bis 5 befindet.

7. Verfahren zur Herstellung eines Kassettenmoduls gemäß einem der Ansprüche 1 bis 5, **dadurch gekennzeichnet, dass** die Verbindung der Membran (5, 6) zu einer weiteren Komponente des Kassettenmoduls mittels DurchstrahlSchweißen mit einem Laser erfolgt, wobei die Erwärmung der einen das Laserlicht absorbierenden Schicht der Membran (5, 6) erfolgt.

8. Verfahren nach Anspruch 7, **dadurch gekennzeichnet, dass** die Verbindung der Membran (5, 6) zu einer weiteren Komponente in dem Zustand erfolgt, in dem die Membran (5, 6) in den Grundkörper eingesetzt ist.

## Claims

1. A cassette module for controlling fluid flows and for use in blood treatment systems or in infusion systems, comprising:
a base body having means for the flow guidance (70, 70') of a fluid flow, in particular of a liquid flow, and
two membranes (5, 6) which are at least sectionally in contact with the base body, wherein
an actuation element (3) is arranged between the membranes (5, 6) which is configured to act on the means for the flow guidance (70, 70'),
the cassette module has a first functional layer (7) which comprises means for the flow guidance (70, 70') of a fluid flow and a second functional layer (2) which is arranged between the two membranes (5, 6),
**characterized in that**
the cassette module has a third functional layer (1) which has means for actuating (12, 12') one or both membranes (5, 6).

2. A cassette module in accordance with claim 1, **characterized in that** the means for actuating (12, 12') the membrane (5, 6) are pneumatic means or hydraulic means, in particular chambers or channels, in which excess pressure or underpressure is present or can be generated.

3. A cassette module in accordance with one of the preceding claims, **characterized in that** the means for the flow guidance (70, 70') of a fluid flow has one or more channels, valve sections and pump sections.

4. A cassette module in accordance with one of the preceding claims, **characterized in that** at least one, a plurality of or all functional layers (7, 2, 1) comprise flexurally stiff material, preferably plastic.

5. A cassette module in accordance with one of the preceding claims, **characterized in that** the membrane or membranes (5, 6) is/are elastic membranes (5, 6).

6. A blood treatment machine or infusion machine, having a slot in which a cassette module in accordance with one of the claims 1 to 5 is located.

7. A method of manufacturing a cassette module in accordance with one of the claims 1 to 5, **characterized in that** the connection of the membrane (5, 6) to a further component of the cassette module takes place by means of transmission welding using a laser, wherein the heating of the one layer of the membrane (5, 6) absorbing the laser light takes place.

8. A method in accordance with claim 7, **characterized in that** the connection of the membrane (5, 6) to a further component takes place in the state in which the membrane (5, 6) is inserted into the base body.

## Revendications

1. Module cassette destiné à la commande de flux de fluide et à l'utilisation dans des systèmes de traitement du sang ou des systèmes de perfusion, comprenant :
un corps de base doté de moyens destinés au guidage de l'écoulement (70, 70') d'un flux de fluide, en particulier d'un flux de liquide, et
deux membranes (5, 6), qui sont au moins en partie en liaison avec le corps de base, dans lequel
un élément de commande (3), qui est conçu pour agir sur les moyens destinés au guidage de l'écoulement (70, 70'), est disposé entre les membranes (5, 6) et
le module cassette comporte une première couche fonctionnelle (7), qui comprend des moyens destinés au guidage de l'écoulement (70, 70') d'un flux de fluide, et une deuxième couche fonctionnelle (2), qui est disposée entre les deux membranes (5, 6),
**caractérisé en ce que**
le module cassette comporte une troisième couche fonctionnelle (1), qui comporte des moyens destinés à l'actionnement (12, 12') d'une ou des deux membranes (5, 6).

2. Module cassette selon la revendication 1, **caractérisé en ce que** les moyens destinés à l'actionnement (12, 12') de la membrane (5, 6) sont des moyens pneumatiques ou hydrauliques, en particulier des chambres ou des canaux, dans lesquels une surpression ou une dépression règne ou peut être générée.

3. Module cassette selon l'une des revendications précédentes, **caractérisé en ce que** les moyens destinés au guidage de l'écoulement (70, 70') d'un flux de fluide comportent un ou plusieurs canaux, segments de valves et segments de pompe.

4. Module cassette selon l'une des revendications précédentes, **caractérisé en ce qu'**au moins une, plusieurs ou toutes les couches fonctionnelles (7, 2, 1) sont composées d'un matériau rigide en flexion, de préférence de matière plastique.

5. Module cassette selon l'une des revendications précédentes, **caractérisé en ce que** la ou les membranes (5, 6) sont des membranes élastiques (5, 6).

6. Machine de traitement du sang ou de perfusion comprenant un logement, dans lequel se trouve un module cassette selon l'une des revendications 1 à 5.

7. Procédé de fabrication d'un module cassette selon l'une des revendications 1 à 5, **caractérisé en ce que** la liaison de la membrane (5, 6) avec un autre composant du module cassette est effectuée par soudage par transmission au moyen d'un laser, le chauffage de la couche de la membrane (5, 6) qui absorbe la lumière laser étant effectué.

8. Procédé selon la revendication 7, **caractérisé en ce que** la liaison de la membrane (5, 6) avec un autre composant est effectuée dans l'état dans lequel la membrane (5, 6) est placée dans le corps de base.
